# EUROPÄISCHE PATENTSCHRIFT

(11) **EP 1 721 583 B1**
(45) Veröffentlichungstag und Bekanntmachung des Hinweises auf die Patenterteilung: **20.04.2016**
(21) Anmeldenummer: 06016301.1
(22) Anmeldetag: 14.11.2001
(51) Int. Cl.: A61F 2/28, A61F 2/44, A61F 2/46, A61F 2/30

(54) **Implantat zum Einsetzen zwischen Wirbelkörper sowie Operationsinstrument zum Betätigen des Implantats**
Implant for the insertion between the vertebraes and operation tool for using the implant
Implant pour poser entre les vertèbres et outil d'opération pour l' utilisation de l'implant

(30) Priorität: 27.12.2000 DE 10065232
(43) Veröffentlichungstag der Anmeldung: 15.11.2006
(62) Teilanmeldung aus: 01126995.8
(73) Patentinhaber: Ulrich GmbH & Co. KG, 89081 Ulm (DE)
(72) Erfinder: Neumann, Carsten, Dr. med., 93007 Bad Abbach (DE)
(74) Vertreter: Hentrich Patentanwälte PartG mbB

(56) Entgegenhaltungen:
- EP-A1- 0 188 954
- WO-A-01/72246
- DE-A- 4 423 257
- US-A- 5 236 460

## Beschreibung

Die Erfindung betrifft ein Implantat zum Einsetzen zwischen Wirbelkörper mit den Merkmalen gemäß dem Oberbegriff des Anspruchs 1. Die Erfindung betrifft weiterhin ein Implantat und ein Operationsinstrument zum Einsetzen des Implantats zwischen Wirbelkörper.

Ein Implantat der eingangs genannten Art ist beispielsweise aus der DE 44 23 257 Al bekannt, das sich in der Praxis sehr gut bewährt hat und mit dem sehr gute Heilungserfolge nach der Implantation erzielt werden konnten. Während der Implantation bei der Operation an der offenen Wirbelsäule hat es sich allerdings als teilweise problematisch gezeigt, daß zum Verschrauben des ersten

Implantatteils gegenüber dem zweiten Implantatteil ein großes Operationsfeld benötigt wird, da mittels Schwenkbewegungen die gegenseitige Verdrehung bewirkt werden muß. Platz für derartige Schwenkbewegungen steht aber nicht in jedem Fall ausreichend zur Verfügung, insbesondere wenn im Sinne einer minimal-invasiven Chirurgie nur kleine Zugänge zur Wirbelsäule gelegt werden.

Der Erfindung liegt daher die Aufgabe zugrunde, ein Implantat der eingangs genannten Art so auszubilden, daß eine Längenänderung des Implantats auch bewirkt werden kann, ohne daß dazu ein gegenseitiges Verschrauben des ersten und des zweiten Implantatteils erforderlich ist.

Diese Aufgabe wird bei einem Implantat der eingangs genannten Art nach der Erfindung durch die Merkmale aus dem kennzeichnenden Teil des Patentanspruchs 1 gelöst.

Dieses Implantat bietet den großen Vorteil, daß aufgrund der Kegelradverzahnung eine Verdrehung des zweiten Implantatteils durch ein rein radial an das Implantat angesetztes Operationsinstrument bewirkt werden kann allein durch dessen Verdrehen. Damit ist zur Längenänderung ein im wesentlichen punktförmiger Zugang zum Implantat ausreichend, wodurch eine allein zum Zwecke der Distraktion vergrößerte Wunde vermieden ist.

Nach einer bevorzugten Ausführungsform der Erfindung ist vorgesehen, daß das erste Implantatteil und das zweite Implantatteil als rohrförmige Hülsen gestaltet sind, und daß das erste Implantatteil mit dem Gewindering das zweite Implantatteil außenseitig umfaßt. Diese Gestaltung der Erfindung unter Verwendung zweier Hülsen bietet den Vorteil, daß der Innenraum des Implantats frei ist und genutzt werden kann, um beispielsweise Knochenzement oder patienteneigene oder fremde Knochenspäne einzufüllen, um das Implantat zuverlässig einwachsen zu lassen. Um bei der Längenänderung des Implantats bei dem Verschrauben des zweiten Implantatteils gegenüber dem ersten Implantatteil eine Klemmung bzw. Selbsthemmung des Gewindes zu vermeiden, ist das Implantat so gestaltet, daß im zweiten Implantatteil mindestens ein Führungsschlitz zur Aufnahme eines dem ersten Implantatteil zugeordneten Stiftes ausgebildet ist. Zum Zwecke einer verbesserten Führung hat sich dabei bewährt, wenn der Führungsschlitz und der Stift zweifach in diametraler Anordnung vorgesehen sind.

Um die Betätigung des Gewinderinges mittels eines Operationsinstrumentes zu vereinfachen, ist in der Wandung des hülsenförmigen ersten Implantatteiles mindestens ein Durchbruch vorhanden, der den dem Gewindering zugeordneten Rand schneidet. Dieser Durchbruch kann als ein Lager für das an das Implantat anzusetzende Operationsinstrument genutzt werden.

Um die Führung des Operationsinstrumentes nicht auf die Dicke der Hülsenwand beschränken zu müssen, sind fluchtend mit dem Durchbruch im zweiten Implantatteil zwei Langlöcher vorhanden. Diese Langlöcher ermöglichen auch, daß gegenüberliegend zum Durchbruch in der Wandung des ersten Implantatteils eine Gewindebohrung ausgebildet ist, die von dem Durchbruch dann erreichbar ist.

Im Hinblick auf ein schnelleres Einwachsen des Implantats hat es sich weiterhin als günstig erwiesen, wenn am zweiten Implantatteil an dem dem ersten Implantatteil abgewandten Ende ein Kranz ausgebildet ist. Dieser Kranz vergrößert die Anlagefläche des zweiten Implantatteils an dem Wirbelkörper. Ist der Kranz mit Abstand vom freien Ende des zweiten Implantatteils angeordnet, so kann er als Sicherung für einzubringenden Knochenzement dienen und zugleich das Gewinde gegen das Zusetzen durch Knochenzement schützen. Alternativ ist es gleichfalls möglich, daß in dem Kranz parallel zur Längsachse des Implantats verlaufende Löcher ausgebildet sind.

Um eine möglichst große Führungslänge des Gewinderinges auf dem Gewinde zu erreichen, ist das Implantat so gestaltet, daß der Gewindering am Innenumfang einen Ringbund aufweist.

Um eine Sicherung der Lage des Gewinderinges gegenüber dem ersten Implantatteil bewirken zu können, ist das erfindungsgemäße Implantat weiterhin derartig gestaltet, daß das erste Implantatteil eine in der rohrförmigen Hülse ausgebildete, senkrecht zur Längsachse des Implantats verlaufende Quernut aufweist. Zweckmäßigerweise liegt diese Quernut parallel zur Verbindungsachse des Durchbruchs und der Gewindebohrung. Günstig ist weiterhin, wenn die Quernut beidseits der Verbindungsachse in der rohrförmigen Hülse des ersten Implantatteils ausgebildet ist.

Durch das Operationsinstrument gemäß den Merkmalen aus dem kennzeichnenden Teil des Patentanspruches 15 kann durch die radial von dem Implantat wegstehende Welle, die senkrecht zur Längsachse des Implantats orientiert ist, allein durch Verdrehung um die Längsachse der Welle die Kegelradverzahnung des Ritzels verdreht werden, das bei dem Zusammenwirken mit der Kegelradverzahnung des Gewinderinges die zur Längenänderung des Implantats erforderliche Drehung des Gewinderinges bewirkt.

Zur leichteren Handhabung des Operationsinstruments während der Operation ist vorgesehen, daß die Welle drehbar in einem Handgriff gelagert und zum Antrieb mit einem Rändelrad an dem dem Ritzel abgewandten Ende versehen ist.

Eine besonders bevorzugte Ausführungsform des Operationsinstruments ist dadurch gekennzeichnet, daß die Welle als Hohlwelle gestaltet ist, in der ein gegenüber der Hohlwelle verdrehbarer Stab gelagert ist, der das Ritzel mit seinem freien Ende durchgreift und an dem ein zu der Gewindebohrung korrespondierendes Stabgewinde ausgebildet ist. Dies bietet den Vorteil, daß die Lage des ersten Implantatteils gegenüber dem Operationsinstrument durch das Stabgewinde gesichert werden kann, so daß durch die Ausübung des notwendigen Druckes, um die Kegelradverzahnung des Ritzels in Eingriff mit der Kegelradverzahnung des Gewinderinges zu bringen, eine Lageänderung des bereits zwischen den Wirbelkörpern der Wirbelsäule plazierten Platzhalters nicht provoziert wird.

Eine weitere bevorzugte Ausführungsform der Erfindung ist dadurch gekennzeichnet, daß die Welle in einer an dem Handgriff befestigten Hülse angeordnet ist, die an dem dem Ritzel zugeordneten Ende eine Gabel mit einer Sicherung der gegenseitigen axialen Lage des Gewinderinges und des ersten Implantatteils bewirkenden Gabelfingern aufweist. Als günstig hat sich dabei erwiesen, wenn die Gabel insgesamt vier das Implantat haltende Gabelfinger aufweist, von denen zwei zu einem ersten Gabelpaar zusammengefaßt und zur Anlage auf dem Gewindering und zwei zu einem zweiten Gabelpaar zusammengefaßt und zum Einführen in die Quernuten vorgesehen sind. Mit einem derartigen Operationsinstrument kann das Implantat sehr sicher erfaßt und an einem vorbestimmten Ort gehalten werden, wobei trotz der erforderlichen Verdrehung der Welle zur Längenänderung keine Gefahr besteht, daß das Implantat im Operationsfeld verrutscht oder das erste Implantat und das zweite Implantat sich gegeneinander verschieben und so eine Längenerstreckung einnehmen, die nicht durch eine Verdrehung des Gewinderinges bewirkt wird und durch diesen abgesichert ist.

Im Hinblick auf verbesserte hygienische Verhältnisse bei der Desinfektion ist es zweckmäßig, wenn die Welle mit dem Rändelrad, der Stab und der Handgriff mit der Hülse zerlegbar montiert sind.

Im folgenden soll die Erfindung an einem in der Zeichnung dargestellten Ausführungsbeispiel näher erläutert werden; es zeigen:
- Fig. 1: eine perspektivische Darstellung des erfindungsgemäßen Implantates bei der Betätigung durch das erfindungsgemäße Operationsinstrument,
- Fig. 2: eine perspektivische Darstellung des Implantats isoliert,
- Fig. 3: eine perspektivische Darstellung des zweiten Implantatteils,

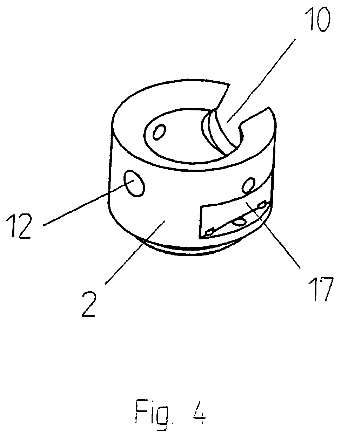

- Fig. 4: eine perspektivische Darstellung des ersten Implantatteils,

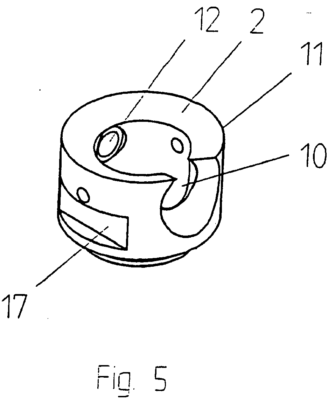

- Fig. 5: eine perspektivische Darstellung des ersten Implantatteils aus einer gegenüber Fig. 4 anderen Sicht,
- Fig. 6: eine Seitenansicht des zweiten Implantatteils,

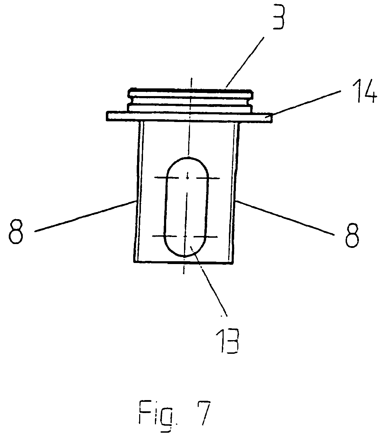

- Fig. 7: eine Seitenansicht des zweiten Implantatteils mit gegenüber Fig. 6 um 90° gedrehter Darstellung,
- Fig. 8: einen Querschnitt durch den die Kegelradverzahnung aufweisenden Gewindering,
- Fig. 9: das in seine Einzelteile zerlegte, erfindungsgemäße Operationsinstrument in einer Draufsicht,
- Fig. 10: eine perspektivische Darstellung des Operationsinstrumentes komplett montiert und in seine Einzelteile zerlegt,

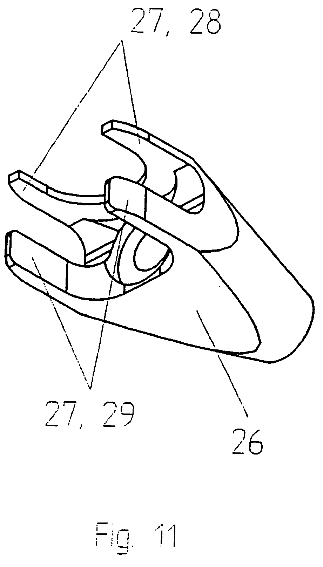

- Fig. 11: eine perspektivische Darstellung der Gabel der Hülse und

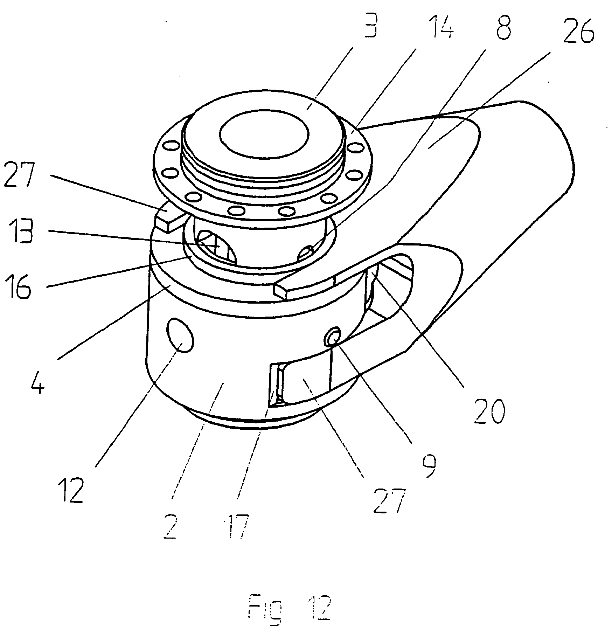

- Fig. 12: die Detaildarstellung XII aus Fig. 1.

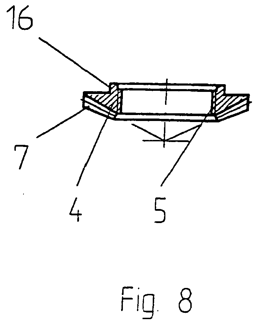

Das in der Zeichnung dargestellte Implantat 1 dient zum Einsetzen zwischen in der Zeichnung selber nicht dargestellte wirbelkörper der Wirbelsäule als Platzhalter für aus der Wirbelsäule entfernte Wirbel oder Wirbelteile. Dieses Implantat 1 weist ein erstes Implantatteil 2 und ein zweites Implantatteil 3 auf, die in Richtung ihrer koaxialen Längsachse gegeneinander verstellbar sind, um im Sinne einer Distraktion der Wirbelkörper eine Längenänderung bewirken zu können. Das erste Implantatteil 2 und das zweite Implantatteil 3 sind als rohrförmige Hülsen gestaltet, wobei das erste Implantatteil 2 das zweite Implantatteil 3 außenseitig umfaßt. Dem ersten Implantatteil 2 ist ein drehbarer Gewindering 4 zugeordnet, der an seiner Innenumfangsfläche ein Ringgewinde 5 aufweist, mit dem dieser in ein dem zweiten Implantatteil 3 zugeordnetes Gewinde 6 eingreift. An der Außenumfangsfläche ist der Gewindering 5 mit einer Kegelradverzahnung 7 versehen (Fig. 8).

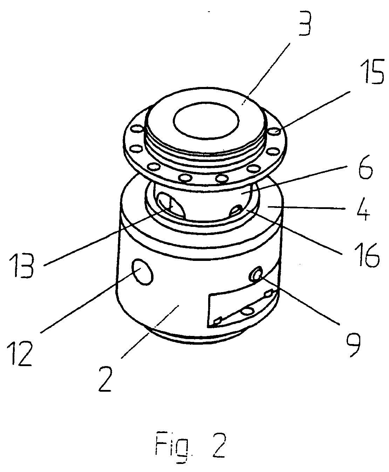

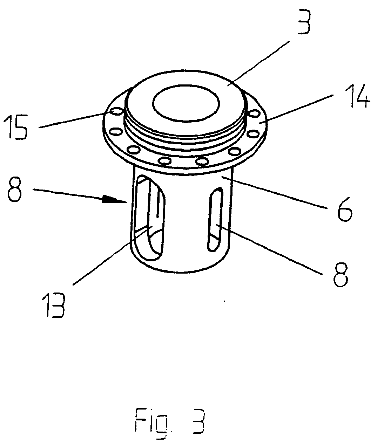

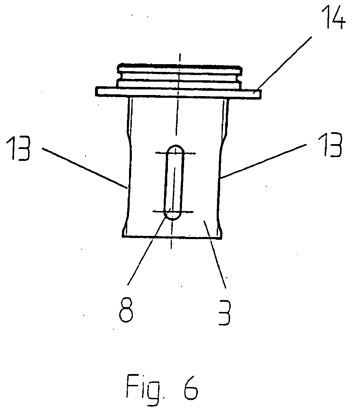

Wie insbesondere aus den Figuren 3 und 6 zu erkennen ist, sind im zweiten Implantatteil 3 zwei einander gegenüberliegende Führungsschlitze 8 ausgebildet, in die dem ersten Implantatteil 2 zugeordnete Stifte 9 hineinragen (Fig. 2).

Neben diesen Stiften 9 weist das erste Implantatteil 2 weiterhin einen Durchbruch 10 auf, der den dem Gewindering 4 zugeordneten Rand 11 schneidet und auf einer Geraden mit einer auf der gegenüberliegenden Seite ausgebildeten Gewindebohrung 12 liegt. Fluchtend sind mit dem Durchbruch 10 in dem zweiten Implantatteil 3 zwei Langlöcher 13 vorgesehen, durch die hindurch die Gewindebohrung 12 in jedweder Höhenlage des zweiten Implantatteils 3 gegenüber dem ersten Implantatteil 1 erreichbar ist.

An dem zweiten Implantatteil 3 ist mit Abstand von dessen freien Ende auf der dem ersten Implantatteil 2 abgewandten Seite ein Kranz 14 ausgebildet, in dem parallel zur Längsachse des Implantats 1 verlaufende Löcher 15 ausgebildet sind.

Der Gewindering 4 weist am Innenumfang einen Ringbund 16 auf, während das erste Implantatteil 2 eine in der rohrförmigen Hülse ausgebildete, senkrecht zur Längsachse des Implantats 1 verlaufende Quernut 17 aufweist, die parallel zur Verbindungsachse des Durchbruchs 10 und der Gewindebohrung 12 liegt, wobei bei dem in der Zeichnung dargestellten Ausführungsbeispiel die Quernut 17 zweifach beidseits der Verbindungsachse vorgesehen ist.

Der am Gewindering 4 ausgebildete Ringbund 16 sowie die Quernuten 17 dienen zur besseren Ausrichtung und Bestimmung der Lage eines nachfolgend näher zu beschreibenden Operationsinstrumentes 18, das zur Handhabung und Betätigung des Implantats 1 bei der Längenverstellung dient.

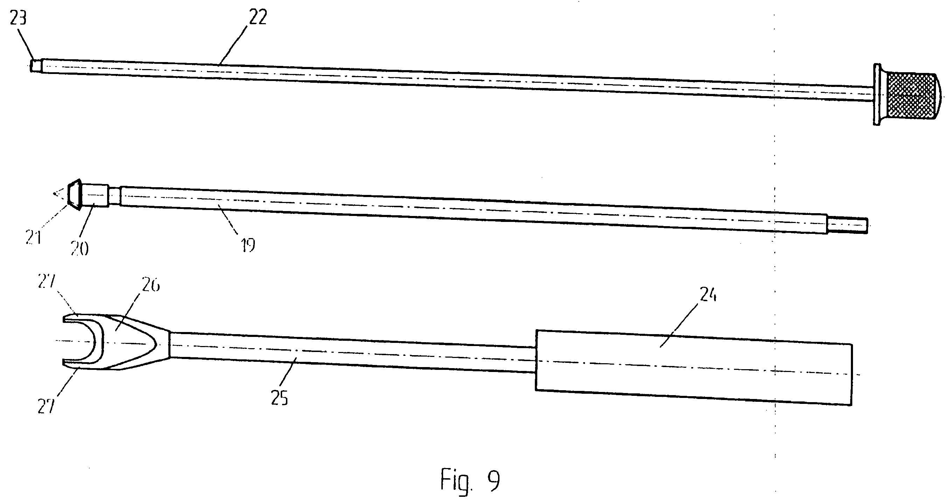

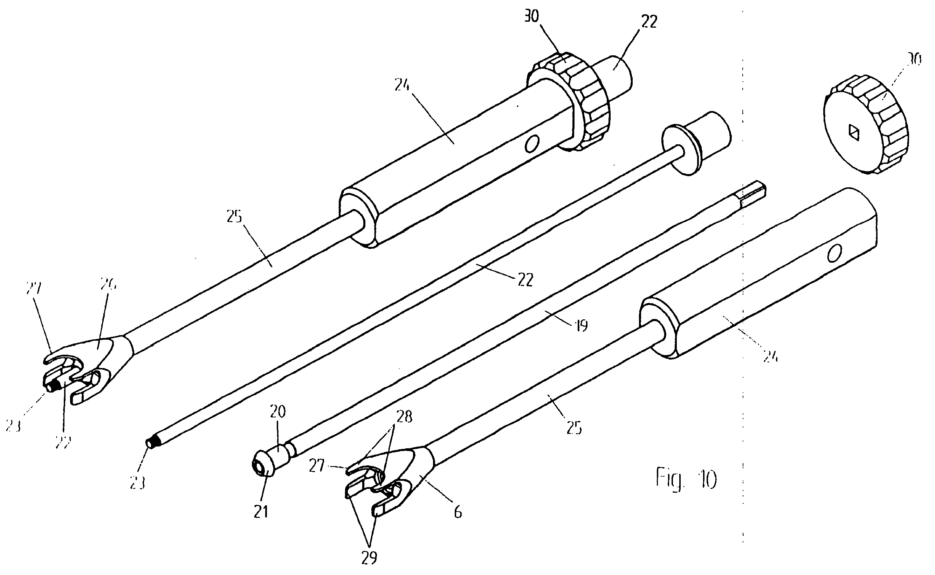

Das Operationsinstrument 18 weist dazu an dem freien Ende einer Welle 19 ein zur Wellenachse koaxiales Ritzel 20 auf. An diesem Ritzel 20 ist eine Kegelradverzahnung 21 ausgebildet (Fig. 9, Fig. 10). Die Welle 19 selber ist als Hohlwelle gestaltet, in der ein Stab 22 gegenüber der Hohlwelle verdrehbar gelagert ist. Dieser Stab 22 durchgreift das Ritzel 20 und weist an seinem freien Ende ein zu der Gewindebohrung 12 korrespondierendes Stabgewinde 23 auf. Die Hohlwelle selber ist drehbar in einem Handgriff 24 gelagert, der dazu eine Hülse 25 aufweist, in die die Hohlwelle eingeführt ist. Diese Hülse 25 besitzt an dem dem Ritzel 20 zugeordneten Ende eine Gabel 26 mit vier Gabelfingern 27, von denen zwei zu einem ersten Gabelpaar 28 zusammengefaßt und zwei zu einem zweiten Gabelpaar 29 zusammengefaßt sind.

Nachdem vorstehend der Aufbau des Implantats 1 und des Operationsinstrumentes 18 geschildert ist, soll nachfolgend deren Zusammenwirken detaillierter dargelegt werden.

Zum Plazieren des erfindungsgemäßen Implantats 1 als Platzhalter zwischen zwei Wirbelkörpern wird zunächst das Implantat 1 auf die Gabel 26 gesteckt, wobei das zweite Gabelpaar 29 in die beiden Quernuten 17 eingeführt wird und das erste Gabelpaar 28 auf dem Gewindering 4 und radial innen an dem Ringbund 16 zur Anlage kommt, so daß eine axiale Verschiebung des Gewinderinges 4 in der Längsachse des Implantats 1 gegenüber dem ersten Implantatteil 2 blockiert ist. Anschließend wird der Stab 22 mit dem Stabgewinde 23 durch den Durchbruch 10 des ersten Implantatteils 2 sowie die beiden Langlöcher 13 des zweiten Implantatteils 3 geführt und in die Gewindebohrung 12 des ersten Implantatteils 2 eingeschraubt, so daß das Operationsinstrument 18 nicht mehr in radialer Richtung von dem Implantat 1 abgezogen werden kann, die Lage des Operationsinstrumentes 18 damit insgesamt gegenüber dem Implantat 1 gesichert ist. Durch das Einschrauben des Stabgewindes 23 in die Gewindebohrung 12 wird zugleich die Kegelradverzahnung 21 des Ritzels 20 in Eingriff mit der Kegelradverzahnung 7 des Gewinderinges 4 gebracht, so daß bei der nachfolgenden Verdrehung der Welle 19 um ihre Wellenachse durch ein Rändelrad 30 eine Verdrehung des Gewinderinges 4 um die zur Wellenachse um 90° gedrehte Längsachse des Implantats 1 erfolgt.

Nach der korrekten Plazierung und Distraktion des Implantats 1 kann das Operationsinstrument 18 in sinngemäßer Umkehrung der vorstehend beschriebenen Schritte wieder von dem Implantat 1 gelöst werden, das durch die dann frei zugänglichen Führungsschlitze 8, Langlöcher 13 und die Gewindebohrungen 12 sowie den Durchbruch 10 genügend Zugangsmöglichkeiten in das Innere der hülsenförmigen ersten Implantatteile 2 und zweiten Implantatteile 3 bietet, um Knochenzement oder autologes oder homologes Knochenmaterial in das Implantat einzubringen.

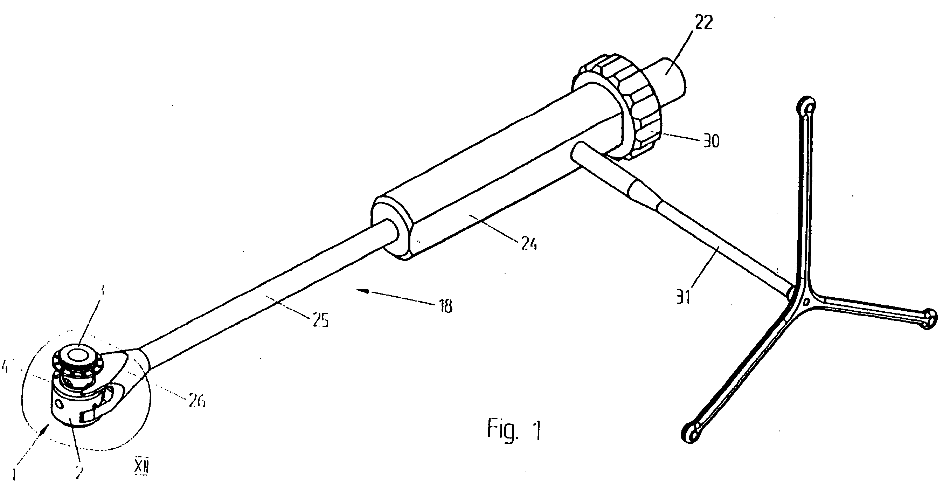

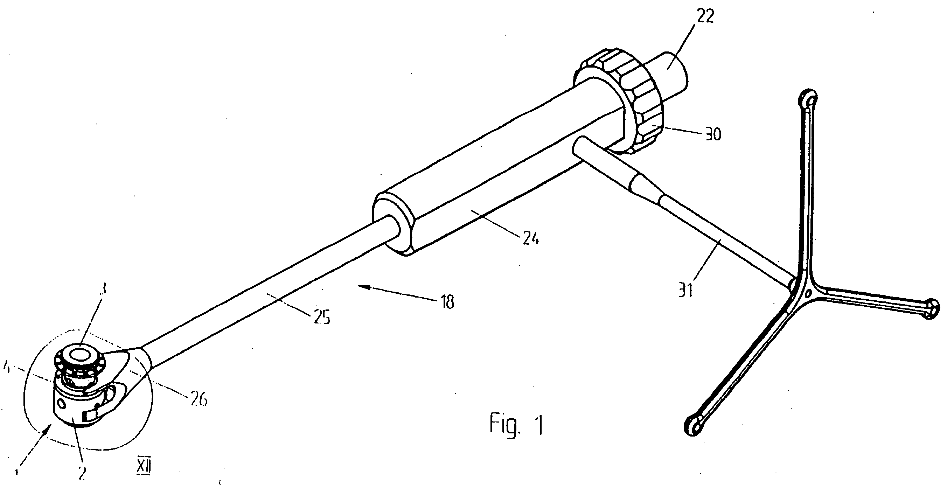

Der in Figur 1 dargestellte mit dem Bezugszeichen 31 bezeichnete Aufsatz dient der Kopplung des Operationsinstruments 18 mit Positionsgebern für die Computer-Navigation.

## Patentansprüche

1. Implantat zum Einsetzen zwischen Wirbelkörper der Wirbelsäule als Platzhalter für aus der Wirbelsäule entfernte Wirbel oder Wirbelteile, mit einem ersten Implantatteil (2) und einem zweiten Implantatteil (3), die in Richtung ihrer koaxialen Längsachse zur Längenänderung des Implantats (1) gegeneinander verstellbar sind, **dadurch gekennzeichnet, daß** dem ersten Implantatteil (2) ein drehbarer Gewindering (4) zugeordnet ist, der zur Längenänderung mit einem Ringgewinde (5) in ein dem zweiten Implantatteil (3) zugeordnetes Gewinde (6) eingreift, und daß im zweiten Implantatteil (3) mindestens ein Führungsschlitz (8) zur Aufnahme eines dem ersten Implanttatteil (2) zugeordneten Stiftes (9) ausgebildet ist, um bei der Längenänderung eine Führung zu bieten.

2. Implantat nach Anspruch 1, **dadurch gekennzeichnet, daß** das erste Implantatteil (2) und das zweite Implantatteil (3) als rohrförmige Hülsen gestaltet sind, und daß das erste Implantatteil (2) mit dem Gewindering (4) das zweite Implantatteil (3) außenseitig umfaßt.

3. Implantat nach einem der Ansprüche 1 und 2, **dadurch gekennzeichnet, daß** der Führungsschlitz (8) und der Stift (9) zweifach in diametraler Anordnung vorgesehen sind.

4. Implantat nach einem der Ansprüche 2 bis 3, **dadurch gekennzeichnet, daß** in der Wandung des hülsenförmigen ersten Implantatteils (2) mindestens ein Durchbruch (10) vorhanden ist, der den dem Gewindering (4) zugeordneten Rand (11) schneidet.

5. Implantat nach Anspruch 4, **dadurch gekennzeichnet, daß** fluchtend mit dem Durchbruch (10) im zweiten Implantatteil (3) zwei Langlöcher (13) vorhanden sind.

6. Implantat nach Anspruch 5, **dadurch gekennzeichnet, daß** gegenüberliegend zum Durchbruch (10) in der Wandung des ersten Implantatteils (2) eine Gewindebohrung (12) ausgebildet ist.

7. Implantat nach einem der Ansprüche 1 bis 6, **dadurch gekennzeichnet, daß** am zweiten Implantatteil (3) an dem dem ersten Implantatteil (2) abgewandten Ende ein Kranz (14) ausgebildet ist.

8. Implantat nach Anspruch 7, **dadurch gekennzeichnet, daß** der Kranz (14) mit Abstand vom freien Ende des zweiten Implantatteils (3) angeordnet ist.

9. Implantat nach Anspruch 7 oder 8, **dadurch gekennzeichnet, daß** in dem Kranz (14) parallel zur Längsachse des Implantats (1) verlaufende Löcher (15) ausgebildet sind.

10. Implantat nach einem der Ansprüche 1 bis 9, **dadurch gekennzeichnet, daß** der Gewindering (4) am Innenumfang einen Ringbund (16) aufweist.

11. Implantat nach einem der Ansprüche 2 bis 10, **dadurch gekennzeichnet, daß** das erste Implantatteil (2) eine in der rohrförmigen Hülse ausgebildete, senkrecht zur Längsachse des Implantats (1) verlaufende Quernut (17) aufweist.

12. Implantat nach Anspruch 11, **dadurch gekennzeichnet, daß** Quernut (17) parallel zur Verbindungsachse des Durchbruchs (10) und der Gewindebohrung (12) liegt.

13. Implantat nach Anspruch 12, **dadurch gekennzeichnet, daß** die Quernut (17) beidseits der Verbindungsachse in der rohrförmigen Hülse des ersten Implantatteils (2) ausgebildet ist.

14. Implantat nach einem der Ansprüche 1 bis 13, **dadurch gekennzeichnet, daß** der Gewindering mit einer Kegelradverzahnung versehen ist.

15. Implantat nach einer der Ansprüche 1 bis 14, **dadurch gekennzeichnet, daß** ein Operationsinstrument mit einem ein Stabgewinde (23) aufweisenden Stab (22) vorgesehen ist, daß in der Wandung des ersten Implantatteils eine Gewindebohrung (12) ausgebildet ist, zum Einschrauben des Stabgewindes (23), wodurch ein Ritzel (20) einer Welle (19) mit dem Gewindering (4) in Eingriff gebracht wird.

16. Implantat nach Anspruch 15, **dadurch gekennzeichnet, daß** das Ritzel (20) eine Kegelradverzahnung (21) aufweist.

17. Implantat nach Anspruch 15 oder 16, **dadurch gekennzeichnet, daß** die Welle (19) drehbar in einem Handgriff (24) gelagert und zum Antrieb mit einem Rändelrad (30) an dem dem Ritzel (20) abgewandten Ende versehen ist.

18. Implantat nach Anspruch 15 oder 16, **dadurch gekennzeichnet, daß** die Welle (19) als Hohlwelle gestaltet ist, in der der Stab (22) gegenüber der Hohlwelle verdrehbar gelagert ist.

19. Implantat nach Anspruch 17 oder 18, **dadurch gekennzeichnet, daß** die Welle (19) in einer an dem Handgriff (24) befestigten Hülse (25) angeordnet ist, die an dem dem Ritzel (20)
zugeordneten Ende eine Gabel (26) mit eine Sicherung der gegenseitigen axialen Lage des Gewinderinges (4) und des ersten Implantatteils (2) bewirkenden Gabelfingern (27) aufweist.

20. Implantat nach Anspruch 18, **dadurch gekennzeichnet, daß** die Gabel (26) insgesamt vier das Implantat haltende Gabelfinger (27) aufweist, von denen zwei zu einem ersten Gabelpaar (28) zusammengefaßt und zur Anlage auf den Gewindering (4) und zwei zu einem zweiten Gabelpaar (29) zusammengefaßt und zum Einführen in die Quernuten (17) vorgesehen sind.

21. Implantat einem der Ansprüche 15 bis 20, **dadurch gekennzeichnet, daß** die Welle (19) mit dem Rändelrad (30), der Stab (22) und der Handgriff (24) mit der Hülse zerlegbar montiert sind.

## Claims

1. Implant for insertion between the vertebrae of the spinal column as a place holder for vertebrae or parts of vertebrae removed from the spinal column, comprising a first implant part (2) and a second implant part (3) which are adjustable against one another in the direction of their coaxial longitudinal axis in order to change the length of the implant (1), **characterized in that** a rotatable threaded ring (4) is allocated to the first implant part (2), which, for the purpose of changing the length engages with an annular thread (5) into the thread allocated to the second implant part (3), and that, in the second implant part (3), at least one guide slot (8) is formed to accommodate a pin (9) allocated to the first implant part (2) in order to provide a guide when changing the length.

2. Implant according to claim 1, **characterised in that** the first implant part (2) and the second implant part (3) are configured as tubular sleeves, and that the first implant part (2) encompasses the second implant part (3) on the outside with the threaded ring (4).

3. Implant according to any one of claims 1 and 2, **characterised in that** the guide slot (8) and the pin (9) are provided twofold in a diametric arrangement.

4. Implant according to any one of claims 2 to 3, **characterised in that**, arranged in the wall of the sleeve-shaped first implant part (2), is at least one passage aperture (10) which intersects the edge (11) allocated to the threaded ring (4),

5. Implant according to claim 4, **characterised in that**, flush with the passage aperture (10) in the second implant part (3), two longitudinal holes (13) are provided.

6. Implant according to claim 5, **characterised in that**, opposite the passage aperture (10) in the wall of the first implant part (2), a threaded borehole (12) is formed.

7. Implant according to any one of claims 1 to 6, **characterised in that**, at the second Implant part (3), at the end facing away from the first implant part (2), is a rim (14).

8. Implant according to claim 7, **characterised in that** the rim (14) is arranged at a distance spacing from the free end of the second implant part (3).

9. Implant according to claim 7 or 8, **characterised in that** holes (15) are formed in the rim (14) parallel to the longitudinal axis of the implant (1).

10. Implant according to any ane of claims 1 to 9, **characterised in that** the threaded ring (4) comprises a ring composite (16) on the inner circumference.

11. Implant according to any one of claims 2 to 10, **characterised in that** the first implant (2) comprises a transverse groove (17) formed in the tubular sleeve and running perpendicular to the longitudinal axis of the implant (1).

12. Implant according to claim 11, **characterised in that** the transverse groove (17) lies parallel to the connection axis of the passage aperture (10) and the threaded borehole (12).

13. Implant according to claim 12, **characterised in that** the transverse groove (17) is formed on both sides of the connection axis in the tubular sleeve of the first implant part (2).

14. Implant according to any one of claims 1 to 13, **characterised in that** the threaded ring is provided with a bevel gear tooth arrangement.

15. Implant according to any one of claims 1 to 14, **characterised in that** a surgical instrument is provided with a bar (22) comprising a bar thread (23), and that a threaded borehole (12) is formed in the wall of the first implant part for the bar thread (23) to be screwed into, as a result of which a pinion (20) of a shaft (19) is brought into engagement with the threaded ring (4).

16. Implant according to claim 15, **characterised in that** the pinion (20) comprises a bevel gear tooth arrangement (21).

17. Implant according to claim 15 or 16, **characterised in that** the shaft (19) is rotatably mounted in a handle (24) and is provided, for the purposes of a drive movement, with a knurled wheel (30) at the end facing away from the pinion (20).

18. Implant according to claim 15 or 16, **characterised in that** the shaft (19) is configured as a hollow shaft, in which the bar (22) is mounted such as to be rotatable in relation to the hollow shaft.

19. Implant according to claim 17 or 18, **characterised in that** the shaft (19) is arranged in a sleeve (25) secured to the handle (24), which comprises, at the end allocated to the pinion (20), a fork (26), with fork fingers (27) which have the effect of securing the axial position on the opposite side of the threaded ring (4) and of the first implant part (2).

20. Implant according to claim 18, **characterised in that** the fork (26) comprises a total of four fork fingers (27) holding the implant, two of which are brought together to form a first fork pair (28) and for contact on the threaded ring (4) and two are brought together to form a second fork pair and for introduction into the transverse grooves (17).

21. Implant according to any one of claims 15 to 20, **characterised in that** the shaft (19), with the knurled wheel (20), the bar (22), and the handle (24) are mounted with the sleeve such as to be capable of being dismantled.

## Revendications

1. Implant destiné à être inséré entre des corps vertébraux de la colonne vertébrale, en tant qu'élément de substitution de vertèbres ou de parties de vertèbres retirées de la colonne vertébrale, comprenant une première partie d'implant (2) et une deuxième partie d'implant (3), qui peuvent être déplacées l'une par rapport à l'autre dans le sens de leur axe longitudinal coaxial, en vue de modifier la longueur de l'implant (1), **caractérisé en ce qu'**une bague filetée (4) tournante est associée à la première partie d'implant (2), bague qui, en vue de la modification de la longueur, s'engage avec un filetage de bague (5) dans un filetage (6) associée à la deuxième partie d'implant (3), et **en ce qu'**au moins une fente de guidage (8) est ménagée dans la deuxième partie d'implant (3), en vue de recevoir une broche (9) associée à la première partie d'implant (2), afin de fournir un guidage lors de la modification de la longueur.

2. Implant selon la revendication 1, **caractérisé en ce que** la première partie d'implant (2) et la deuxième partie d'implant (3) sont réalisées sous forme de manchons tubulaires, et **en ce que** la première partie d'implant (2) entoure avec la bague filetée (4) la deuxième partie d'implant (3), sur la face externe.

3. Implant selon l'une des revendications 1 et 2, **caractérisé en ce que** la fente de guidage (8) et la broche (9) sont prévues deux fois dans une configuration diamétralement opposée.

4. Implant selon l'une des revendications 2 à 3, **caractérisé en ce qu'**il est prévu, dans la paroi de la première partie d'implant (2) en forme de manchon, au moins un perçage (10) qui coupe le bord (11) associé à la bague filetée (4).

5. Implant selon la revendication 4, **caractérisé en ce que** deux trous oblongs (13) sont prévus dans la deuxième partie d'implant (3), dans l'alignement du perçage (10).

6. Implant selon la revendication 5, **caractérisé en ce qu'**un trou taraudé (12) est réalisé dans la paroi de la première partie d'implant (2), en vis-à-vis du perçage (10).

7. Implant selon l'une des revendications 1 à 6, **caractérisé en ce qu'**une couronne (14) est réalisée sur la deuxième partie d'implant (3), à l'extrémité opposée à la première partie d'implant (2).

8. Implant selon la revendication 7, **caractérisé en ce que** la couronne (14) est disposée à distance de l'extrémité libre de la deuxième partie d'implant (3).

9. Implant selon la revendication 7 ou 8, **caractérisé en ce que** des trous (15) sont réalisés dans la couronne (14), qui s'étendent parallèlement à l'axe longitudinal de l'implant (1).

10. Implant selon l'une des revendications 1 à 9, **caractérisé en ce que** la bague filetée (4) présente un collet annulaire (16) sur la circonférence intérieure.

11. Implant selon l'une des revendications 2 à 10, **caractérisé en ce que** la première partie d'implant (2) présente une rainure transversale (17) réalisée dans le manchon tubulaire et s'étendant perpendiculairement à l'axe longitudinal de l'implant (1).

12. Implant selon la revendication 11, **caractérisé en ce que** la rainure transversale (17) est parallèle à l'axe de liaison du perçage (10) et du trou taraudé (12).

13. Implant selon la revendication 12, **caractérisé en ce que** la rainure transversale (17) est réalisée de part et d'autre de l'axe de liaison dans le manchon tubulaire de la première partie d'implant (2).

14. Implant selon l'une des revendications 1 à 13, **caractérisé en ce que** la bague filetée est pourvue d'une denture de roue conique.

15. Implant selon l'une des revendications 1 à 14, **caractérisé en ce qu'**il est prévu un instrument chirurgical doté d'une tige (22) présentant un filetage de tige (23), **en ce qu'**un trou taraudé (12) est réalisé dans la paroi de la première partie d'implant, en vue du vissage du filetage de tige (23), grâce à quoi un pignon (20) d'un arbre (19) est amené en prise avec la bague filetée (4).

16. Implant selon la revendication 15, **caractérisé en ce que** le pignon (20) présente une denture de roue conique (21).

17. Implant selon la revendication 15 ou 16, **caractérisé en ce que** l'arbre (19) est monté avec possibilité de rotation dans une poignée (24) et, en vue de l'entraînement, est pourvu d'une molette (30) à l'extrémité opposée au pignon (20).

18. Implant selon la revendication 15 ou 16, **caractérisé en ce que** l'arbre (19) est réalisé comme arbre creux dans lequel la tige (22) est montée avec possibilité de rotation par rapport à l'arbre creux.

19. Implant selon la revendication 17 ou 18, **caractérisé en ce que** l'arbre (19) est disposé dans un manchon (25) qui est fixé à la poignée (24) et qui, à l'extrémité associée au pignon (20), présente une fourche (26) avec des doigts de fourche (27) provoquant le verrouillage de la position axiale réciproque de la bague filetée (4) et de la première partie d'implant (2).

20. Implant selon la revendication 18, **caractérisé en ce que** la fourche (26) présente au total quatre doigts de fourche (27) qui portent l'implant et dont deux sont regroupés pour former une première paire de fourche (28) et pour venir en appui sur la bague filetée (4), et deux sont regroupés pour former une deuxième paire de fourche (29) et pour s'engager dans les rainures transversales (17).

21. Implant selon l'une des revendications 15 à 20, **caractérisé en ce que** l'arbre (19) est assemblé de façon démontable avec la molette (30), et la tige (22) et la poignée (24) sont assemblées de manière démontrable avec le manchon.
